# FASCICULE DE BREVET EUROPEEN

(11) **EP 2 237 700 B1**
(45) Date de publication et mention de la délivrance du brevet: **07.06.2017**
(21) Numéro de dépôt: 09720238.6
(22) Date de dépôt: 26.01.2009
(51) Int. Cl.: A45D 20/12

(54) **SILENCIEUX POUR APPAREIL DE SÉCHAGE ET SÈCHE-CHEVEUX SILENCIEUX**
SCHALLDÄMPFER FÜR EINE TROCKNUNGSVORRICHTUNG UND LEISER HAARTROCKNER
SILENCER FOR DRYING APPLIANCE AND QUIET HAIRDRYER

(30) Priorité: 25.01.2008 FR 0800396
(43) Date de publication de la demande: 13.10.2010
(73) Titulaire: Velecta Paramount SAS, 75015 Paris (FR)
(72) Inventeur: GAILLARD, Christophe, F-41200 Romorantin-Lanthenay (FR); GUILLOSSON, Michel, F-45160 Olivet (FR); QUESSARD, Roland, F-41200 Romorantin-Lanthenay (FR)
(74) Mandataire: Debay, Yves
(86) Numéro de dépôt international: PCT/FR2009/000081
(87) Numéro de publication internationale: WO 2009/112690

(56) Documents cités:
- EP-A- 0 631 738
- DE-A1- 3 141 245
- DE-U1- 8 805 910
- JP-A- 60 135 700

## Description

La présente invention concerne le domaine des appareils de séchage et en particulier des sèche-cheveux. La présente invention concerne plus particulièrement un silencieux pour un appareil de séchage tel qu'un sèche-cheveux, ainsi qu'un sèche-cheveux silencieux.

Les séchoirs, en particulier les sèche-cheveux sont généralement équipés d'un ventilateur ou d'une turbine réalisant une fonction de soufflerie. Souvent, la turbine est de type centrifuge, de façon à produire un flux d'air canalisé par des parois d'un carter du séchoir, jusqu'à une buse concentrant le flux d'air pour obtenir une pression efficace pour le séchage. La fonction de séchage est généralement améliorée par la présence d'une résistance en aval du ventilateur ou de la turbine, de manière à chauffer l'air expulsé. L'entrée d'air au niveau de la turbine est généralement pourvue d'une grille destinée à éviter l'accès aux parties tournantes de la turbine et à protéger l'appareil de l'encrassement. Cette grille comporte souvent une première partie fixée sur le pavillon d'admission d'air de la turbine centrifuge et constituée par des nervures espacées de telle sorte qu'elles empêchent au moins la pénétration d'un doigt d'enfant et une seconde partie constituée par un filtre réalisé par un grillage et/ou par une mousse à alvéoles ouvertes et dont la trame est dimensionnée de telle façon qu'elle puisse tamiser les poussières et les cheveux. Souvent, ce filtre est rendu amovible par diverses solutions telles que son insertion dans un capot permettant son démontage et son remontage rapide pour faciliter son nettoyage.

Un problème dans le domaine des appareils de séchage concerne le niveau sonore souvent élevé du moteur et du ventilateur ou de la turbine. En effet, les rotations du moteur et du ventilateur ou de la turbine sont sources de bruit. Le bruit généré par de tels appareils est fonction de la puissance du moteur et de la vitesse de rotation de la turbine. Ce bruit est particulièrement gênant pour l'utilisateur, notamment dans le cas des sèche-cheveux qui sont utilisés à proximité des oreilles des utilisateurs.

Il est connu dans l'art antérieur des solutions pour optimiser le rendement de la turbine centrifuge et atténuer son niveau sonore. Certaines solutions consistent à diminuer les turbulences de l'air générées au voisinage de ses pales en rayonnant le bord de l'admission de l'air en amont ou en aval de la grille et du filtre à poussières. Certaines solutions de l'art antérieur consistent à doter les sèche-cheveux d'écrans destinés à diminuer la propagation des ondes acoustiques rayonnant perpendiculairement à l'admission d'air. Certains écrans sont réalisés en matériau isolants et d'autres se présentent soit sous la forme d'un carter percé d'ouïes d'aspiration latérales, soit d'un capotage entourant le corps de l'appareil au droit de la turbine de telle sorte que cette admission d'air se fasse de façon annulaire entre ce corps et ce capotage. Ces solutions présentent l'inconvénient de ne pas être satisfaisantes. En effet, pour masquer l'effet sonore des turbulences générées au bord d'attaque des pales de la turbine, la section débitante de l'admission d'air doit être diminuée au maximum et cela au détriment du rendement aéraulique de la turbine. L'atteinte de l'objectif visé en débit n'est alors réalisable que par l'augmentation de la vitesse du moteur, ce qui induit une diminution de la durée de vie de l'appareil. Par ailleurs, l'atténuation sonore gagnée est alors en partie perdue par l'augmentation du bruit généré par le moteur. D'autre part, les ouïes d'aspiration latérales ne permettent une diminution satisfaisante du bruit.

Un autre problème dans le domaine des appareils de séchage concerne le fait que l'atténuation du bruit ne peut généralement pas être ajoutée sur des dispositifs déjà distribués.

Dans ce contexte, il est intéressant de proposer un sèche-cheveux silencieux et un silencieux pour appareil de séchage, tel qu'un sèche-cheveux en particulier, permettant de réduire le bruit émis par l'appareil.

Un but de la présente invention est de pallier certains inconvénients de l'art antérieur en proposant un sèche-cheveux silencieux générant peu de bruit. Le document DE 88 05 910 U1 enseigne le préambule des revendications indépendantes de la présente demande.

Ce but est atteint par les revendications indépendantes de la présente demande. D'autres particularités sont exposées dans les revendications dépendantes.

D'autres particularités et avantages de la présente invention apparaîtront plus clairement à la lecture de la description ci-après, faite en référence aux dessins annexés, dans lesquels :
- la figure 1 représente une vue en coupe d'un appareil de séchage silencieux selon un exemple,
- la figure 2 représente une vue en coupe d'un appareil de séchage muni d'un silencieux selon divers modes de réalisation de l'invention,
- la figure 3 représente une vue en coupe d'un appareil de séchage silencieux selon un exemple,
- la figure 4 représente une vue en perspective d'un filtre en matériau poreux équipant les dispositifs selon divers modes de réalisation de l'invention,
- les figures 5A et 5B représentent des vues de face, en transparence, respectivement d'une turbine connue de l'art antérieur et d'une turbine améliorée selon certains modes de réalisation de l'invention,
- les figures 6A et 6B représentent des vues en perspective, d'une turbine améliorée selon certains modes de réalisation de l'invention, respectivement assemblée et en vue éclatée,
- la figure 7 représente une vue en coupe d'un appareil de séchage muni d'un silencieux selon un exemple.

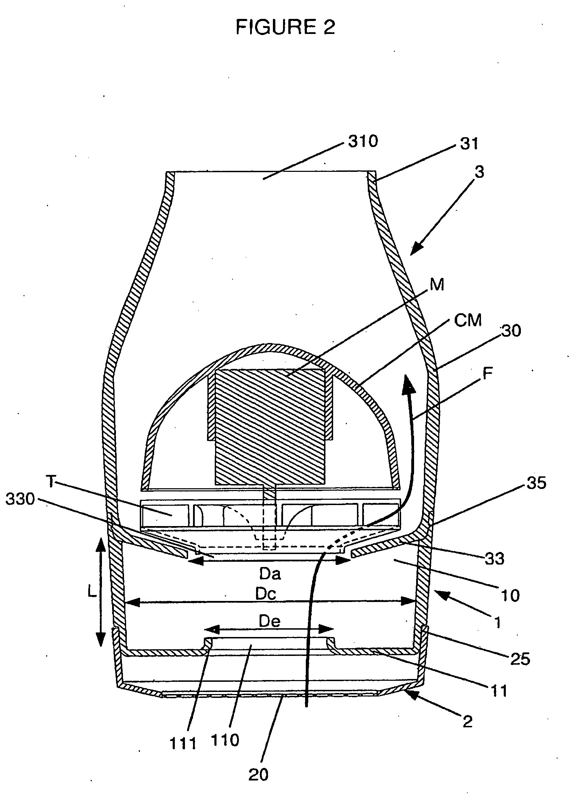

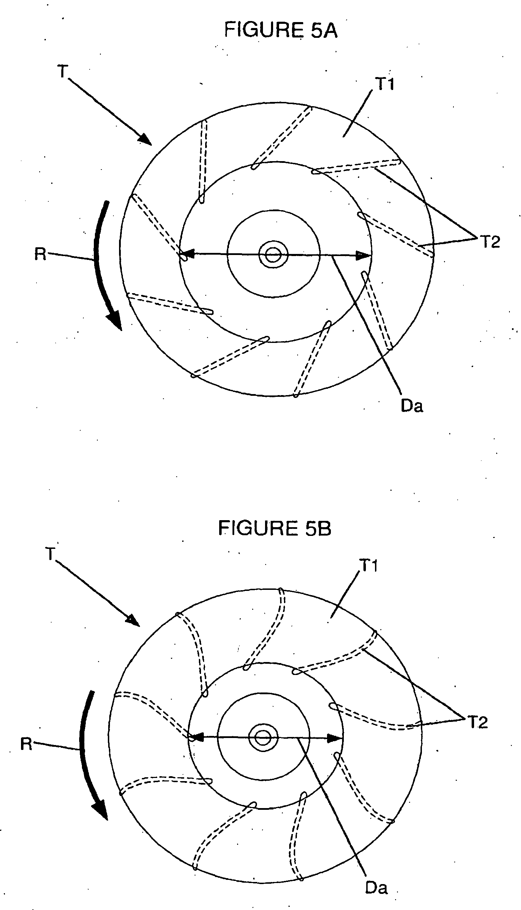

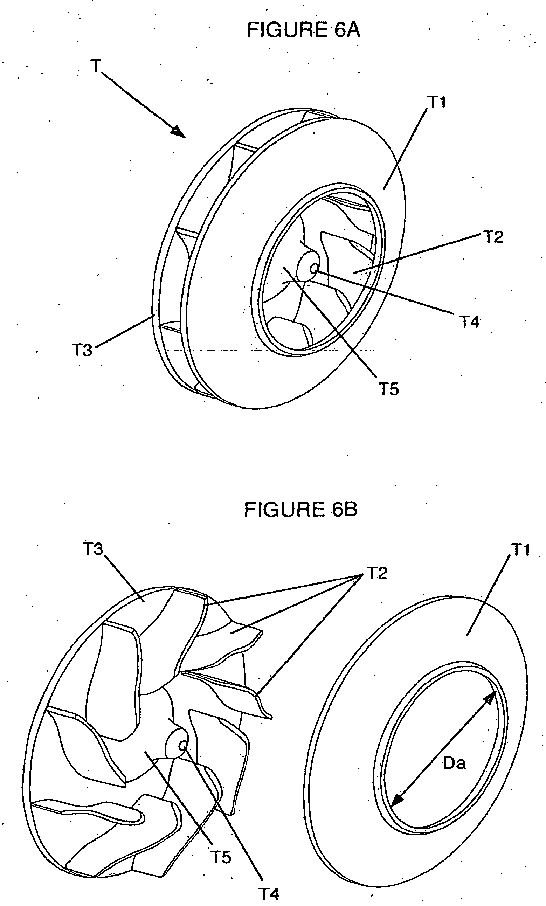

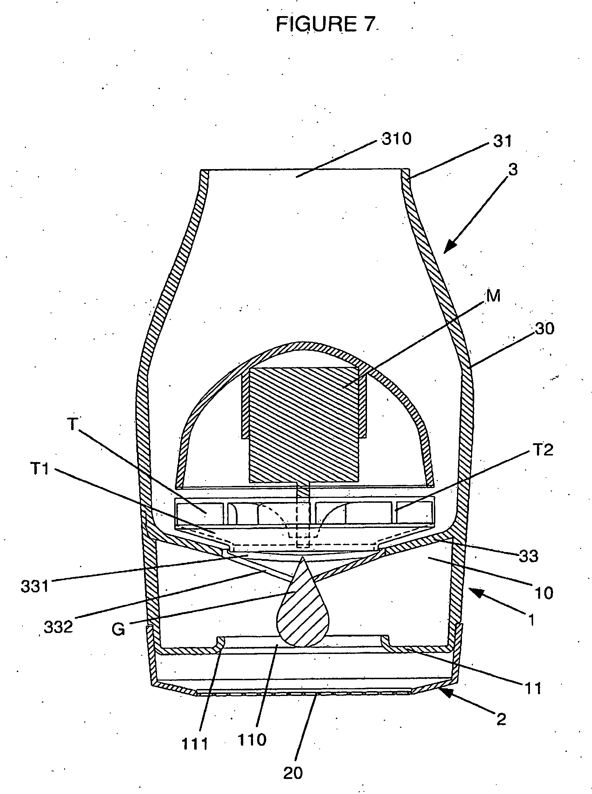

La présente invention concerne un silencieux (1) pour appareils de séchage, en particulier pour sèche-cheveux (3), et un sèche-cheveux silencieux. Par le terme appareils de séchage, ou même le terme sèche-cheveux, on entend ici tout type d'appareil destiné au séchage, qu'il s'agisse de cheveux ou non. En effet, l'invention est adaptée aux dispositifs et appareils expulsant de l'air, chauffé ou non, par exemple pour le séchage de diverses matières telles que, par exemple, des cheveux. Ainsi, l'utilisation qui peut être faite de l'invention n'est pas limitée au séchage des cheveux. L'invention ne sera donc limitée qu'à une utilisation avec un appareil comprenant les moyens décrits ici ou à toute adaptation à la portée de l'homme de métier. L'invention est adaptée à tout appareil comportant au moins un carter (30) comprenant une extrémité d'entrée (33), une extrémité de sortie (31) et au moins un moteur (M) entraînant en rotation au moins une turbine (T) aspirant de l'air par au moins un orifice (330) d'admission au niveau de l'extrémité d'entrée (33) en amont de la turbine (T) et l'expulsant en aval par au moins un orifice (310) de sortie au niveau de l'extrémité de sortie (31). Ce type d'appareil entraîne un flux (F) d'air au travers du carter (30), comme particulièrement visible sur la figure 2. Ce flux (F) d'air aspiré passe par les orifices d'entrée (110) et d'admission (330), à travers le silencieux (1) qui en réduit le bruit comme expliqué ci-après, puis passe par la turbine centrifuge qui l'expulse en périphérie, entre le carter (30) de l'appareil et le carter moteur (CM). Le flux d'air pourra bien entendu être chauffé par un dispositif de chauffage en aval de la turbine, avant sa sortie par l'orifice (310) de sortie. Comme il est connu de l'art antérieur, l'orifice (330) d'admission pourra être muni d'une grille (332, figure 7) empêchant l'insertion d'un doigt d'enfant au niveau de la turbine. Cette grille pourra naturellement avoir des formes diverses et l'exemple de la grille (332) décrit plus loin de la figure 7 n'est qu'illustratif et nullement limitatif. De même, comme connu de l'art antérieur, l'orifice (310) de sortie au niveau de l'extrémité de sortie (31) pourra être habillé d'une buse concentrant le flux d'air pour augmenter la pression en sortie de l'appareil. Dans les exemples de réalisation représentés sur les figures, il s'agit d'une turbine centrifuge créant un flux (F) d'air généré autour de la turbine et du moteur et canalisé par les parois internes du carter (30), mais l'invention n'est pas limitée à ce type d'agencement. D'autre part, la turbine (T) représentée sur les figures et particulièrement visible sur les figures 5B, 6A et 6B est un exemple de réalisation particulièrement avantageux de l'invention mais d'autres types de turbines pourront être utilisées, bien que le type de turbine décrit plus loin en référence aux figures 5B, 6A et 6B soit particulièrement adapté au but principal de l'invention qu'est la réduction de bruit. Enfin, l'orifice (330) d'admission pourra, selon divers modes de réalisation, être ménagé dans une paroi droite au niveau de l'extrémité (33) d'entrée ou dans une paroi tronconique comme représenté sur les figures. On notera ici que l'orifice (330) d'admission est défini par rapport à la paroi (33) de l'extrémité d'entrée mais, d'une manière générale, l'admission concerne l'entrée d'air dans la turbine. Ainsi, comme détaillé plus loin, lorsqu'il s'agit d'une turbine (T) centrifuge comportant un flasque (T1) au niveau de l'admission d'air dans la turbine, comme particulièrement visible sur les figures 5B, 6A et 6B, l'orifice d'admission qui importe pour l'aspiration d'air et pour le bruit généré est en fait l'orifice d'entrée du flasque (T1) de la turbine (T), que cette dernière soit protégée ou non par une paroi (33) de l'extrémité d'entrée. Ainsi, lorsque l'on parle ici du diamètre (Da) d'admission de l'orifice d'admission, l'homme de métier comprendra qu'en fonction des divers modes de réalisation détaillés ci-après, il peut s'agir du diamètre de l'orifice d'entrée du flasque (T1) ou du diamètre de l'orifice (330) d'admission au niveau de la paroi (33) devant la turbine. On notera également que lorsque la turbine (T) comporte un flasque (T1), comme représenté de façon illustrative sur les figures, et que la turbine est protégée par une paroi (33) de l'extrémité d'entrée, le flasque (T1) et la paroi (33) sont généralement agencés pour que l'espace les séparant soit suffisamment petit pour éviter les phénomènes de reflux de l'air sortant de la turbine vers l'extrémité d'entrée (33). De plus, dans ces modes de réalisation où la turbine (T) est protégée par une paroi et comporte un flasque (T1), le diamètre de l'orifice de la paroi et le diamètre de l'entrée du flasque sont sensiblement identiques, les considérations de diamètres étant, comme expliqué précédemment, généralement focalisées sur l'entrée d'air au niveau de la turbine (T). Ainsi, on pourra considérer ici que le diamètre (Da) de l'orifice (330) d'admission correspond au diamètre d'entrée du flasque (T1) de la turbine (T) et les deux termes sont ici utilisés tour à tour pour désigner le diamètre de l'admission d'air dans la plupart des modes de réalisation décrits.

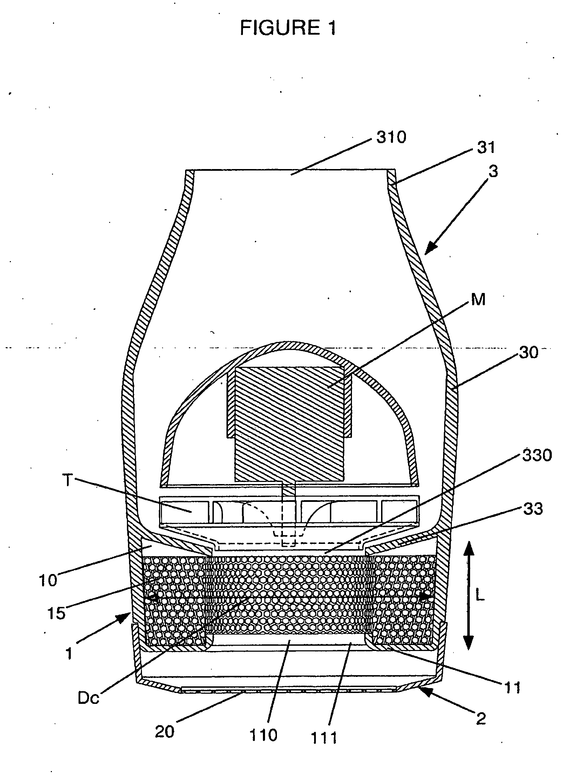

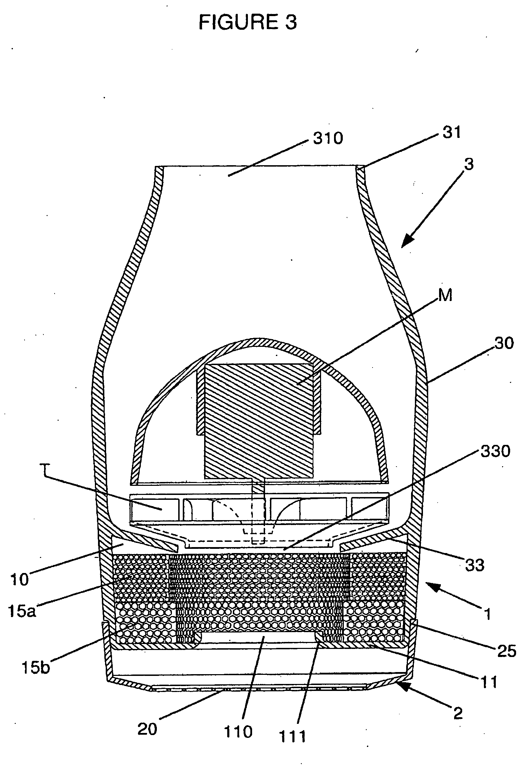

La présente invention concerne un silencieux (1) atténuant les bruits générés par le moteur et la turbine ou le ventilateur, ainsi qu'un sèche-cheveux (3) comportant au moins un silencieux (1). Le silencieux (1) comporte au moins une chambre (10) montée de façon sensiblement étanche en amont de l'orifice (330) d'admission et comportant un orifice (110) d'entrée pour l'aspiration d'air en amont de la chambre (10). Par exemple, cet orifice (110) d'entrée pourra être ménagé dans une paroi (11) fermant partiellement le silencieux. Dans certains modes de réalisation, cette paroi (11) comporte un pavillon (11) dont le bord (111) évasé facilite le glissement de l'air vers l'intérieur de la chambre (10), minimisant ainsi les perturbations. Cette chambre (10) pourra, selon diverses variantes de réalisation être de section cylindrique, tronconique ou polygonale ou de n'importe quelle forme choisie par les concepteurs, bien que les formes cylindriques ou tronconiques soient plus aérodynamiques et donc plus adaptées à l'utilisation qui en est faite. De même, le sèche-cheveux (3), le silencieux (1) ou tous les compartiments qui les composent peuvent avoir naturellement diverses formes bien que les formes cylindriques ou tronconiques soient préférées. Dans la présente description, on entend d'ailleurs par le terme « diamètre » d'une structure la distance (maximum, minimum ou moyenne) séparant deux points situés à l'opposé l'un de l'autre sur la structure en question. En général, dans la présente description, la notion de diamètre est utilisée pour définir des tailles de sections d'écoulement de l'air au travers d'un orifice ou entre deux structures. La section d'écoulement peut bien entendu avoir n'importe quelle forme de pourtour puisque ce pourtour dépend des structures entre lesquelles l'écoulement a lieu ou de l'orifice au travers duquel l'écoulement a lieu. La notion de diamètre étant aisément comprise dans le cas de structures sensiblement circulaires, on étendra ici l'utilisation de ce terme à toute forme que pourraient prendre les éléments de l'invention, tels que par exemple, le sèche-cheveux (3) lui-même, la chambre (10) ou le filtre (15) décrits ici. Dans certains modes de réalisation non conforme à l'invention, le silencieux (1) est réalisé d'un seul tenant avec le carter (30), au niveau de l'extrémité d'entrée (33), comme particulièrement visible sur les figures 1 et 3. Selon l'invention, le silencieux (1) comporte des moyens (35) de fixation sur le carter (30) de l'appareil (3) de séchage, au niveau de l'extrémité d'entrée (33), comme particulièrement visible sur la figure 2. Dans certaines variantes de réalisation, le silencieux est équipé d'un capot (2) en amont de l'orifice (110) d'entrée de la chambre (10). Comme particulièrement visible sur la figure 2, le capot (2) comporte des moyens (25) de fixation sur le silencieux (1), à proximité de l'orifice (110) d'entrée. Ce capot (2) est muni d'au moins un filtre (20). De façon connue en soi, ce filtre pourra comporter une grille et/ou un matériau à alvéoles ouvertes permettant le passage de l'air tout en filtrant les particules. Dans certaines variantes de réalisation, le capot (2) pourra être réalisé d'un seul tenant avec le silencieux (1), le silencieux (1) étant monté amovible sur le carter (30) du sèche-cheveux (3). Dans certains modes de réalisation particulièrement avantageux, le silencieux (1) est destiné à complémenter des sèche-cheveux existants. Dans ces modes de réalisation, le silencieux (1) comporte, d'une part, des moyens (35) de fixation amovible sur le carter (30) identiques à des moyens (25) de fixation amovible du capot (2) sur le carter (30) de l'appareil (3) de séchage, au niveau de l'extrémité d'entrée (33), et d'autre part, à proximité de l'orifice (110) d'entrée, des moyens d'ancrage des moyens (25) de fixation du capot (2), de telle sorte que le silencieux puisse être interposé entre le carter (30) et le capot (2). Ainsi, le silencieux (1) pourra être vendu séparément du sèche-cheveux (3), et même être distribué en complément de sèche-cheveux (3) déjà distribués sur le marché. Par exemple, il existe des sèche-cheveux (3) équipés de capot (2) amovibles vissés ou emboîtés (par un système de type baillonnette) sur l'extrémité d'entrée (33) du sèche-cheveux. Le silencieux pourra alors comporter un filetage destiné à recevoir ce capot (2) une fois dévissé du sèche-cheveux (2) et un taraudage de façon à être vissé à la place du capot (2). Dans un autre exemple, plus pratique d'utilisation, le silencieux pourra comporter des rainures et des tenons pour coopérer, respectivement, avec des tenons du sèche-cheveux et des rainures du capot. Ainsi, l'utilisateur, par une simple manipulation habituelle (lors du nettoyage par exemple) pourra améliorer son sèche-cheveux en lui ajoutant un silencieux (1) conforme à l'invention. L'invention pourra donc prévoir diverses formes et dimensions du silencieux (1) pour qu'il soit adaptable sur divers types de sèche-cheveux (3).

Ainsi, l'invention concerne également un sèche-cheveux (3) muni d'un silencieux selon l'invention. Dans certains modes de réalisation non conforme à l'invention, le silencieux (1) est réalisé d'un seul tenant avec le carter (30), au niveau de l'extrémité d'entrée (33). Selon l'invention, le silencieux (1) est fixé de façon amovible sur le carter (30), au niveau de l'extrémité d'entrée (33). Selon l'invention, le silencieux est destiné à être amovible. Dans ce cas, le sèche-cheveux comporte un capot (2) fixé de façon amovible sur le carter (30), au niveau de l'extrémité d'entrée (33), par des moyens (25) de fixation amovible du capot (2), le silencieux (1) comportant, d'une part, des moyens (35) de fixation amovible sur le carter (30) identiques aux moyens (25) de fixation amovible du capot (2), et d'autre part, à proximité de l'orifice (110) d'entrée, des moyens d'ancrage des moyens (25) de fixation du capot (2), de telle sorte que le silencieux puisse être interposé entre le carter (30) et le capot (2).

De façon plus spécifique, la chambre (10) du silencieux possède un diamètre (Dc) supérieur au diamètre (Da) de l'orifice (330) d'admission (ou, par équivalence, de l'entrée du flasque de la turbine) et au diamètre (De) de l'orifice (110) d'entrée. Ainsi, la chambre offre un volume ouvert autour du flux d'air pour atténuer le bruit. De plus, selon un mode de réalisation de l'invention, le diamètre (De) de l'orifice (110) d'entrée est inférieur au diamètre (Da) de l'orifice (330) d'admission, comme particulièrement visible sur les figures 2 et 3, de façon à faciliter le passage de l'air et minimiser les turbulences dans le silencieux (1) et le sèche-cheveux (3). Cependant, dans certains modes de réalisation, ces deux diamètres (Da, De) seront identiques comme par exemple représenté sur la figure 1 et dans d'autres modes de réalisation, la configuration inverse pourra être envisagée, bien que la configuration représentée sur les figures 2 et 3 soit plus avantageuse car elle minimise le turbulences et permet donc de réduire le bruit. De plus, l'invention prévoit une turbine améliorée détaillée ci-après en référence aux figures 5B, 6A et 6B dont le diamètre d'entrée (Da) est diminué pour minimiser le bruit. Ces modes de réalisation de la turbines détaillés plus loin pourront, notamment et par exemple, être utilisés dans les modes de réalisation où le diamètre (Da) d'admission dans la turbine est sensiblement identique au diamètre (De) d'entrée dans le silencieux. D'autre part, la longueur (L) et le diamètre (Dc) de la chambre (10) sont dimensionnés en fonction des fréquences sonores à atténuer par le silencieux (1) ou de façon à définir un volume accordé en fonction des fréquences sonores à atténuer par le silencieux (1). En effet, la chambre (10) du silencieux (1) selon l'invention comporte un volume entourant le passage ouvert au flux d'air au travers du silencieux qui permet d'atténuer le bruit. Le diamètre (Dc) de la chambre (10) est supérieur au diamètre (Da) de l'orifice (330) d'admission et au diamètre (De) de l'orifice (110) d'entrée, d'une valeur déterminée en fonctions desdites fréquences sonores déterminées. Le Flux (F) d'air traverse la chambre en son centre, sensiblement à l'intérieur d'un cylindre défini par le plus petit diamètre parmi les diamètres (Da, De) de l'orifice (330) d'admission et de l'orifice (110) d'entrée, mais le flux (F) d'air gagne également le reste de la chambre, notamment un volume annulaire, défini entre le diamètre (Dc) de la chambre (10) et au moins un diamètre parmi les diamètres (Da, De) de l'orifice (330) d'admission et de l'orifice (110) d'entrée. Le volume de la chambre et en particulier ce volume annulaire constituent un filtre sonore qui atténue le bruit de l'appareil. En fonction des fréquences sonores à atténuer dans l'appareil sur lequel le silencieux est destiné à être installé, le volume de la chambre devra avoir des dimensions particulières qui seront obtenues par diverses combinaisons du diamètre (Dc) de la chambre et sa longueur (L). En particulier, le sèche-cheveux génère un flux d'air (F) par la rotation de la turbine. Cette rotation et ce flux génèrent un bruit qui est gênant pour l'utilisateur. Il est possible de mesurer et quantifier ce bruit pour déterminer les fréquences sonores qui le composent. Une étude détaillée du spectre des fréquences sonores émises par des sèches-cheveux en fonctionnement permet de déterminer que les fréquences sonores émises dépendent majoritairement de la vitesse de rotation et du nombre de pales de la turbine. Par exemple, une turbine centrifuge comportant 9 pales et ayant une vitesse de rotation comprise entre 12 000 et 20 000 tr/min, génère des fréquences sonores comprises entre 9 x 12 000 / 60 = 1 800 Hz et 20 000 x 9 / 60 = 3 000 Hz. En général, le spectre des fréquences émises par les sèche-cheveux est en fait plus large que cette gamme obtenue directement par ce calcul sur la période de rotation d'une pale. Par exemple, une telle turbine générera en fait un spectre très étalé mais les mesures présentées ici n'ont été réalisées que sur un spectre de fréquences comprises entre 250 Hz et 16000 Hz. De plus, si l'on considère seulement les fréquences les plus audibles (par exemple celles supérieures à 55 décibels audibles, dBA), le spectre est réduit à des fréquences comprises entre 400 Hz et 8000 Hz, les fréquences en-dehors de cette gamme étant beaucoup moins audibles (surtout celles supérieures à 6300 Hz).

Une mesure réalisée avec un micro situé à 1 mètre d'un sèche-cheveux comportant une turbine centrifuge ayant un diamètre d'entrée (« d'admission ») de l'ordre de 35mm, un diamètre de sortie de l'ordre de 64mm, comportant 9 pales et tournant à 13500 tours par minutes (tr/min) génère une pression comprise entre 500 et 1500 Pa et un débit d'air compris entre 0,00833 m³/s et 0,0277m³/s (lorsqu'il est muni de la buse à son extrémité pour concentrer le flux d'air) et un bruit dont le spectre de fréquences est compris (pour les fréquences d'intensité acoustique supérieure à 55dBA) entre 400 Hz et 80000 Hz, avec des fréquences d'intensité acoustique les plus fortes situées entre 1600 Hz et 2500 Hz.

L'invention propose de mettre, en amont de l'admission, un silencieux comportant une chambre dimensionnée, en longueur et en diamètre, pour atténuer les fréquences sonores émises par le sèche-cheveux. Le silencieux utilisé lors de ces mesures possède un diamètre d'entrée de 0,029 m, c'est-à-dire inférieur au diamètre d'admission (0,035 m), de manière à tranquilliser le flux. Le flux, ainsi moins perturbé, génère déjà moins de bruit, mais il apparaît qu'en fonction du dimensionnement de la chambre, cette dernière permet d'atténuer les fréquences sonores émises. Le flux d'air passe au centre de la chambre offrant un volume autour du passage du flux qui permet d'atténuer le bruit. Ce volume autour de la section de passage du flux (entre l'entrée de la chambre et l'admission de la turbine, définies par leurs diamètres respectifs) est particulièrement avantageux par la réduction de bruit qu'il permet mais également par le fait qu'il limite faiblement le débit (moins de 10% de pertes). En effet, ce silencieux limite peu les pertes de débit (de l'ordre de 6% dans le cas d'une turbine classique) et l'on verra plus loin que ces pertes de débit peuvent être encore réduites avec l'utilisation d'une turbine améliorée décrite ci-après (pertes par exemple de l'ordre de 3,5% seulement). En revanche, des chambres comportant des parois telles que des chicanes ou des hélicoïdes donnent des pertes de débit qui sont de l'ordre de 15%. Dans l'exemple du sèche-cheveux donné ci-dessus, les dimensions de la chambre permettant une atténuation efficace des fréquences par le silencieux sont une longueur comprise entre 0,020 m et 0,060 m (unité S.I.), de préférence entre 0,030 m et 0,050 m, et un diamètre compris entre 0,040 m et 0,090 m, de préférence entre 0,050 m et 0,080 m. Diverses combinaisons de diamètres et de longueur sont envisagées mais il ressort des mesures effectuées que pour une longueur donnée, il existe une gamme de diamètres optimaux et, pour un diamètre donné, il existe une gamme de longueurs optimales. Il apparaît donc que les valeurs moyennes du volume résultant de ces combinaisons est important pour l'atténuation et que le volume de la chambre doit être accordé sur les fréquences sonores du sèche-cheveux en accordant à la fois sa longueur et son diamètre. Par exemple, lors des mesures effectuées avec les paramètres décrits ci-dessus, il a pu être déterminé que, pour une longueur donnée, par exemple une longueur optimale de 0,034 m, si le diamètre (et donc le volume) est trop petit, (par exemple un diamètre de 0,054 m, les fréquences sonores moyennes du sèche-cheveux (de l'ordre de 500 à 8000 Hz, et en particulier celles entre 630 et 6300 Hz qui sont les plus audibles par l'utilisateur), ne sont pas bien atténuées. Pour la même longueur (par exemple 0,034 m), lorsque le diamètre (et donc le volume) est trop grand (par exemple un diamètre de 0,079 m), certaines gammes de fréquences basses (de l'ordre de 500 à 1000 Hz) ne sont pas bien atténuées, voire même amplifiées. Dans cet exemple, un compromis est trouvé avec un diamètre de 0,074 m. De même, pour un diamètre donné (par exemple le diamètre de 0,074 m), si la longueur (et donc le volume) est trop petite (par exemple 0,020 m), les fréquences sonores moyennes du sèche-cheveux ne sont pas bien atténuées et certaines fréquences sont même amplifiées, alors que si la longueur (et donc le volume) est trop importante, certaines gammes de fréquences basses ne sont pas bien atténuées, voire même amplifiées. En conclusion, il ressort que la chambre doit avoir un volume moyen qui est correctement accordé (compris entre 0,080 L et 0,220 L, avec une valeur optimale autour de 0,15 L) et résultant d'un compromis entre la longueur (comprise entre 0,030 et 0,040 mm, de préférence 0,034 m) et le diamètre (compris entre 0,070 et 0,080 m, de préférence 0,074m). La forme et les dimensions de la chambre du silencieux sont donc déterminantes dans l'atténuation.

Dans cet exemple des mesures décrites ci-dessus, le sèche-cheveux sans silencieux avait une intensité acoustique de l'ordre de 68 dBA. L'ajout d'un silencieux avec un volume accordé (optimal, avec une longueur de l'ordre de 0,034 m et un diamètre de l'ordre de 0,074 m) réduisait cette intensité acoustique à environ 63 dBA. Dans cet exemple, des volumes de chambre trop petits, par exemple avec un diamètre de 0,054 m et une longueur de 0,034 m ou avec un diamètre de 0,074 m et une longueur de 0,020 m, ne réduisait cette intensité acoustique qu'à 64,8 dBA et 65,5 dBA respectivement. De même, dans cet exemple, des volumes de chambre trop grands, par exemple avec un diamètre de 0,079 m et une longueur de 0,034 m ou avec un diamètre de 0,074 m et une longueur de 0,045 m, ne réduisait cette intensité acoustique qu'à 64,7 dBA et 63,5 dBA, respectivement, et amplifiait certaines fréquences (aux alentours, respectivement, de 1000 et 500 Hz). On notera que la longueur doit être suffisante pour que la chambre forme un volume permettant d'amortir le bruit, par exemple une longueur supérieure à 0,020 m.

Le spectre des fréquences sonores émises par un sèche-cheveux peut également dépendre indirectement de la taille de la turbine. En effet, la taille de la turbine (caractérisée principalement par les diamètres en entrée et en sortie de la turbine) influe principalement sur les caractéristiques de débit et de pression du sèche-cheveux. Comme on souhaite généralement obtenir un sèche-cheveux dont les caractéristiques de débit et de pression sont satisfaisants (par exemple un débit compris entre 0,00833 m³/s et 0,0277m³/s, de préférence entre 0,0138 et 0,0194 m³/s, et une pression comprise entre 500 et 1500 Pa, de préférence entre 700 et 1300Pa), une augmentation de la vitesse de rotation de la turbine compensera (en termes de débit et de pression) une diminution de la taille de la turbine, ou une augmentation de la taille de la turbine compensera (en termes de débit et de pression) une diminution de la vitesse de rotation. Ainsi, pour conserver des caractéristiques de débit et de pression constants, on compense les changements de taille de la turbine par des changements de sa vitesse de rotation. Le spectre des fréquences sonores émises dépendant de la vitesse de rotation, il dépend ainsi indirectement de la taille de la turbine si l'on modifie la vitesse de rotation pour préserver les performances du sèche-cheveux.

Ainsi, on comprendra que les valeurs de longueur et de diamètres optimaux donnés ci-dessus à titre d'exemple sont valables pour le sèche-cheveux décrit, comportant une turbine centrifuge ayant un diamètre « d'admission » de l'ordre de 0,035 m, un diamètre de sortie de l'ordre de 0,064 m (et diamètre d'entrée du silencieux à 0,029 m), comportant 9 pales et tournant à 13500 tr/min, générant une pression comprise entre 700 et 1400 Pa et un débit d'air compris entre 0,0138 et 0,0194 m³/s. On comprendra donc également qu'en fonction des valeurs choisies pour les diamètres d'admission et de sortie de la turbine, on adaptera éventuellement la vitesse de rotation de la turbine pour préserver les performances du sèche-cheveux et on ajustera les dimensions de la chambre du silencieux selon l'invention pour trouver les valeurs optimales (correctement accordées comme expliqué ci-dessus). De même selon le nombre de pales, les ajustements seront faits en conséquence. Cependant, les valeurs optimales données ici, c'est-à-dire une longueur comprise entre 0,020 et 0,050 m, de préférence entre 0,030 et 0,040 m, et un diamètre compris entre 0,040 et 0,090 m, de préférence entre 0,070 et 0,080 m, donnent une chambre qui est capable d'atténuer la plupart des fréquences sonores émises par les sèches-cheveux dans les gammes de fréquences mesurées ici (entre 250 et 16000Hz) et en particulier dans les plus audibles (entre 400 et 8000 Hz).

Certains modes de réalisation de l'invention concernent donc un sèche-cheveux (3) silencieux comportant au moins un carter (30) comprenant une extrémité d'entrée (33), une extrémité de sortie (31) et au moins un moteur (M) entraînant en rotation au moins une turbine (T) aspirant de l'air par au moins un orifice (330) d'admission en amont de la turbine (T) et l'expulsant par au moins un orifice (31) de sortie en aval, en générant des fréquences sonores déterminées, le sèche-cheveux comportant un silencieux (1) comprenant au moins une chambre (10) montée de façon sensiblement étanche en amont de l'orifice (330) d'admission et comportant un orifice (110) d'entrée pour l'aspiration d'air en amont de la chambre (10), le diamètre (Dc) de la chambre (10) étant compris entre 0,040 et 0,090 m, et la longueur (L) étant comprise entre 0,020 et 0,050 m. Dans ces modes de réalisation, la longueur (L) et le diamètre (Dc) de la chambre (10) pourront avoir été dimensionnés de façon à être accordées (ou à définir un volume accordé) en fonction desdites fréquences sonores déterminées du sèche-cheveux.

Parmi ces modes de réalisation de l'invention, certains comporteront un silencieux avec les valeurs préférées (décrites précédemment) de diamètre (Dc) et de longueur (L) de la chambre: de préférence entre 0,070 et 0,080 m pour le diamètre et de préférence entre 0,030 et 0,040 m pour la longueur (L).

Parmi ces modes de réalisation de l'invention, certains comporteront un silencieux avec les valeurs optimales (décrites précédemment) de diamètre (Dc) et de longueur (L) de la chambre: 0,074 m pour le diamètre et 0,034 m pour la longueur (L).

De plus, l'ajout de filtres en matériau poreux dans la chambre peut encore améliorer les propriétés d'atténuation du bruit. Dans l'exemple décrit ci-dessus, avec l'optimum de dimensions de la chambre (longueur de 0,034 m et diamètre de 0,074 m), l'ajout d'un filtre en matériau poreux (de préférence à alvéoles ouvertes) permet de réduire encore le bruit d'environ 1,6dbA. On obtient donc un sèche-cheveux générant de l'ordre de 61-62dBA. On notera que cette atténuation du bruit peut encore être améliorée en ajustant l'indice de porosité du filtre en fonction des fréquences émises et des dimensions de la chambre. De plus, plusieurs filtres d'indices différents peuvent être combinés pour optimiser l'atténuation, comme décrit ci-après.

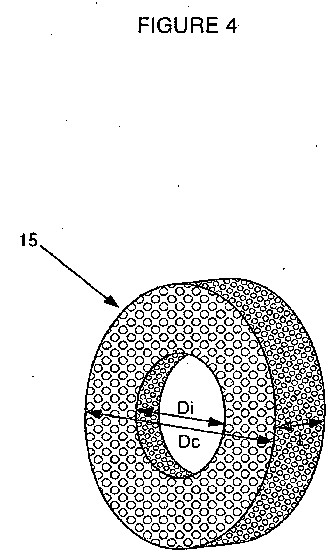

Dans certains modes de réalisation particulièrement avantageux, la chambre (10) comporte au moins un filtre (15) de forme annulaire, comme représenté sur la figure 4. Ce filtre annulaire rempli le volume annulaire de la chambre ouvert autour du passage du flux (F) d'air entre les orifices d'entrée et d'admission et il permet d'atténuer encore mieux le bruit. Comme représenté sur la figure 4, ce filtre possède sensiblement la forme d'un anneau (la section pouvant varier de forme en fonction de la section du silencieux), avec un diamètre externe sensiblement égal au diamètre de la chambre (Dc) et un diamètre intérieur (Di), pour le passage du flux (F) d'air, sensiblement égal à au moins un diamètre parmi les diamètres (Da) de l'orifice (330) d'admission et de l'orifice (110) d'entrée. On notera au passage que, dans certains modes de réalisation, le diamètre (Di) intérieur du (ou des) filtre(s) (15, 15a, 15b) pourra ne pas être constant sur toute la longueur (L), par exemple dans le cas où les diamètres de l'orifice d'entrée et de l'orifice d'admission ne sont pas identiques. Ainsi, le diamètre intérieur du filtre pourra suivre le profil tronconique décrit pas les diamètres de ces deux orifices. De même que précisé précédemment, la forme exacte des sections des divers éléments pourra varier selon diverses variantes de réalisation et les termes relatifs aux diamètres ou à la conicité de la chambre ou du diamètre intérieur du filtre (entre autres) sont utilisés pour illustrer des variantes mais l'homme de métier appréciera les adaptations qui peuvent être faites à partir des formes illustrées ici, comme par exemple des sections carrées ou rectangulaires, avec en particulier des parois inclinées pour suivre le profil décrit par les diverses tailles des divers orifices. De préférence, ce filtre (15) est réalisé en matériau poreux pour permettre la circulation de l'air dans le matériau. De plus, le matériau poreux présent dans le ou les filtre(s) (15, 15a, 15b) est de préférence un matériau à alvéoles ouvertes (ou à alvéoles semi-ouvertes ou tout intermédiaire). Par exemple, le filtre pourra comporter une mousse en polyuréthane ou autre matériau plastique, avec des alvéoles ouvertes (c'est-à-dire communiquant entre elles de façon à faciliter la circulation d'air au travers du matériau). Le volume annulaire et l'indice de porosité du filtre (15) sont accordés en fonction des fréquences sonores à atténuer par le silencieux (1). Ainsi, de même que lorsque la chambre (10) est vide et atténue diverses fréquences en fonction de son volume, comme dans le mode de réalisation illustré sur la figure 2, le filtre (15) atténue diverses fréquences en fonction du volume annulaire qu'il remplit, mais l'atténuation est ici également fonction de l'indice de porosité du filtre (15). Dans certaines variantes de réalisation, la chambre (10) comporte plusieurs filtres (15a, 15b) de densités différentes. En particulier, dans une variante préférée, la densité est croissante en direction de l'orifice (330) d'admission de la turbine (T), comme particulièrement visible sur la figure 3 représentant un exemple de réalisation comportant 2 filtres. Dans d'autres variantes de réalisation, un seul et même filtre à densité variable pourra être envisagé. De préférence, cette densité du filtre sera croissante en direction de l'orifice (330) d'admission. D'autres variantes de réalisation comportant un seul filtre de densité constante mais de forme sensiblement tronconique de telle sorte qu'une partie du filtre est plus comprimée que le reste à l'intérieur de la chambre, ce qui résulte en une densité variable du filtre à l'intérieur de la chambre. De plus, selon diverses variantes de réalisation, les filtres pourront être séparés ou non d'une paroi. Ces filtres (15a, 15b) sont également de forme annulaire et comportent au moins un matériau poreux. De même que précédemment, le volume annulaire et les indices de porosité des filtres (15) sont accordés en fonction des fréquences sonores à atténuer par le silencieux (1). Dans diverses variantes, le ou les filtre(s) (15, 15a, 15b) remplit (ou remplissent) sensiblement le volume annulaire de la chambre (10), comme représenté sur les figures 2 et 3, mais une configuration intermédiaire pourra être envisagée, avec un volume annulaire vide et un ou plusieurs volume(s) annulaire(s) comportant au moins un filtre.

La figure 5A représente une turbine (T) centrifuge connue de l'art antérieur. Cette turbine (T) est munie d'un flasque (T1) recouvrant les pales (T2) et conduisant l'air aspiré entre les pales vers le pourtour de la turbine. Les pales (T2) sont, de façon connue en soi orientée vers l'intérieur de la turbine, dans le sens de rotation (R) de la turbine. Cette orientation des pales est telle qu'entre 2 pales, la section d'écoulement au centre la turbine est inférieure à la section d'écoulement en périphérie. Le flasque (T1) comporte en son centre un orifice d'admission d'un diamètre déterminé. Comme mentionné précédemment, ce diamètre sert de référence pour l'admission d'air et sera défini ici comme le diamètre (Da) d'admission d'air. D'autres types de turbines connues de l'art antérieur ne comporte pas de flasque mais une paroi située en amont joue le même rôle et limite l'entrée d'air à un diamètre (Da) d'admission d'un orifice d'admission de cette paroi, comme mentionné précédemment. Ainsi, on utilisera ici le terme de diamètre (Da) d'admission pour définir cette notion générale d'admission d'air dans la turbine, qu'elle soit munie d'un flasque ou non (voire même qu'elle soit centrifuge ou non). Ce type de turbine de l'art antérieur présente l'inconvénient de générer du bruit. On prévoit donc d'améliorer l'appareil de séchage selon l'invention en y installant une turbine améliorée du type de celle représentée sur les figures 5B, 6A et 6B. Dans cette turbine (T) améliorée selon l'invention, le diamètre (Da) d'admission a été réduit (comme visible par comparaison des figures 5A et 5B), de façon à améliorer l'atténuation, par le silencieux, du bruit généré. On notera que sur les figures, le bord d'attaque des pales (leur extrémité centrale) dépasse à l'intérieur de l'orifice central du flasque. Cependant, cette réduction de diamètre provoque une réduction du débit d'air. Pour conserver une puissance aéraulique satisfaisante, dans certains modes de réalisation, l'extrémité périphérique des pales (T2) pourra être courbée en direction du sens de rotation (R) de la turbine. Ainsi, la pression générée par la turbine (T) est augmentée et compense la perte de débit résultant de la réduction du diamètre d'admission. Dans certaines variantes de réalisation (non représentées), l'extrémité centrale des pales (T2) pourra également être courbée, mais en direction du sens inverse de celui de la rotation (R) de la turbine (T). Ces modes de réalisation de la turbine améliorée, comportant un diamètre (Da) d'admission réduit et une courbure au moins de l'extrémité périphérique des pales (T2) permet d'améliorer l'atténuation, par le silencieux, du bruit généré, tout en conservant une puissance aéraulique satisfaisante. Ainsi, la présente invention ayant pour but de réduire le bruit généré, un appareil de séchage comportant un silencieux et une telle turbine améliorée, optimisant les performances du sèche-cheveux et l'atténuation sonore, fait également l'objet de la présente invention. La figure 6A montre une vue en perspective de la turbine (T) améliorée selon l'invention. Cette turbine (T) comporte un axe (T4) par lequel elle est entraînée en rotation et cet axe est muni d'un moyeu (T5) de forme sensiblement tronconique, ou ayant sensiblement la forme d'une corolle, une tulipe ou un pavillon de profil courbe et évasé vers le bord d'attaque des pales, permettant de guider le flux d'air aspiré vers les pales (T2). Les pales (T2) sont montées sur un disque (T3) fermant la face de la turbine située du côté du moteur. Comme particulièrement visible sur la figure 6B, les pales (T2) de la turbine améliorée du sèche-cheveux selon certains modes de réalisation de l'invention, sont recourbées pour améliorer la pression en sortie de la turbine. Ainsi, l'air aspiré se trouve poussé vers les pales (T2) par le moyeu (T5) interne, puis guidé dans la section d'écoulement croissante entre les pales (T2) flasques, et poussé par l'extrémité périphérique courbe des pales (T2) permettant une meilleure pression.

La figure 7, non conforme à l'invention, représente un dernier perfectionnement de l'invention, qui sera apporté au silencieux et/ou au sèche-cheveux selon divers modes de réalisation de l'invention. Ce perfectionnement consiste en l'adjonction, dans le silencieux, en amont de l'orifice d'admission, d'une goutte (G) aérodynamique réduisant les perturbations aux abords de l'entrée de la turbine (T). Cette goutte (G) pourra, comme représenté sur la figure 7 avoir sensiblement la forme d'une goutte d'eau, c'est-à-dire comporter une portion sensiblement sphérique et, en direction de l'entrée de la turbine, une portion sensiblement conique. Cette goutte sera centrée par rapport à l'orifice d'admission (et/ou à l'orifice d'entrée de la turbine). Dans certaines variantes de réalisation, la portion sphérique sera en matériau lisse tandis que la portion conique sera en matériau poreux (en mousse par exemple). D'autres variantes plus simples dans lesquelles les deux portions sont en matériau identique pourront naturellement être envisagées. Dans l'exemple représenté sur la figure 7, cette goutte est fixée à l'aide de pattes (332). Par exemple, comme mentionné précédemment, l'orifice d'admission peut être muni d'une grille (331, figure 7) comportant des pattes ou des nervures (332) empêchant la pénétration d'un doigt (d'enfant idéalement). Ces nervures (332) pourront donc former une fixation de la goutte (G) en amont de l'admission. Cette fixation pourra être envisagée qu'il existe un grille ou non à ce niveau et d'autres types de fixation pourront bien entendu être adaptés, notamment en minimisant les turbulences aux abords de la turbine. On notera que, comme particulièrement visible sur la figure 7, le diamètre (De) de l'orifice d'entrée a été augmenté pour compenser l'obstruction de l'orifice d'entrée (110) par la goutte (G).

Ainsi, on comprend que les diverses variantes de réalisation permettent d'obtenir un appareil (3) de séchage très silencieux grâce à la présence du silencieux (1), d'une turbine (T) silencieuse et d'une goutte (G) en amont de l'admission de la turbine, le silencieux (1) pouvant être muni de matériau absorbant les sons. On comprend aisément que l'invention prévoit de nombreux modes de réalisation dont des exemples sont décrits ici et que ces exemples sont illustratifs et peuvent être combinés entre eux à moins qu'il ne soit évident qu'ils soient incompatibles. Ainsi, la portée de l'invention s'étend à diverses combinaisons des variantes décrites et aux adaptations évidentes qui sont à la portée de l'homme de métier.

Il doit être évident pour les personnes versées dans l'art que la présente invention permet des modes de réalisation sous de nombreuses autres formes spécifiques sans l'éloigner du domaine d'application de l'invention comme revendiqué. Par conséquent, les présents modes de réalisation doivent être considérés à titre d'illustration, mais peuvent être modifiés dans le domaine défini par la portée des revendications jointes, et l'invention ne doit pas être limitée aux détails donnés ci-dessus.

## Revendications

1. Sèche-cheveux (3) silencieux comportant au moins un carter (30) comprenant une extrémité d'entrée (33), une extrémité de sortie (31) et au moins un moteur (M) entraînant en rotation au moins une turbine (T) aspirant de l'air par au moins un orifice (330) d'admission en amont de la turbine (T) et l'expulsant par au moins un orifice (31) de sortie en aval, le sèche-cheveux comportant un silencieux (1) comprenant au moins une chambre (10) montée de façon sensiblement étanche an amont de l'orifice (330) d'admission et comportant un orifice (110) d'entrée pour l'aspiration d'air en amont de la chambre (10),
- le diamètre (Dc) de la chambre (10) étant supérieur au diamètre (Da) de l'orifice (330) d'admission et au diamètre (De) de l'orifice (110) d'entrée ;
**caractérisé en ce que**:
- le sèche-cheveux (3) comporte un capot (2) apte à être fixé de façon amovible sur le carter (30), au niveau de l'extrémité d'entrée (33) ;
- le silencieux (1) est apte à être fixé de façon amovible sur le carter (30), au niveau de l'extrémité d'entrée (33), grâce à des moyens (35) de fixation amovible sur le carter (30) identiques à des moyens (25) de fixation amovible du capot (2) du sèche-cheveux (3);
- le silencieux comporte, d'une part, des moyens (35) de fixation amovibles sur le carter (30) identiques aux moyens (25) de fixation amovibles du capot (2), et d'autre part, à proximité de l'orifice (110) d'entrée, des moyens d'ancrage des moyens (25) de fixation du capot (2), de telle sorte que le silencieux puisse être interposé entre le carter (30) et le capot (2).

2. Sèche-cheveux (3) silencieux comportant au moins un carter (30) comprenant une extrémité d'entrée (33), une extrémité de sortie (31) et au moins un moteur (M) entraînant en rotation au moins une turbine (T) aspirant de l'air par au moins un orifice (330) d'admission en amont de la turbine (T) et l'expulsant par au moins un orifice (31) de sortie en aval, le sèche-cheveux comportant un silencieux (1) comprenant au moins une chambre (10) montée de façon sensiblement étanche an amont de l'orifice (330) d'admission et comportant un orifice (110) d'entrée pour l'aspiration d'air en amont de la chambre (10),
- le diamètre (Dc) de la chambre (10) étant supérieur au diamètre (Da) de l'orifice (330) d'admission et au diamètre (De) de l'orifice (110) d'entrée ;
**caractérisé en ce que** :
- le sèche-cheveux (3) comporte un capot (2) apte à être fixé de façon amovible sur le carter (30), au niveau de l'extrémité d'entrée (33) ;
- le silencieux (1) comporte un capot réalisé d'un seul tenant avec le silencieux ;
- le silencieux (1) est apte à être fixé de façon amovible sur le carter (30), au niveau de l'extrémité d'entrée (33), en remplacement du capot (2) du sèche-cheveux (3), par des moyens (35) de fixation amovible du silencieux (1).

3. Sèche-cheveux (3) silencieux selon une des revendications 1 à 2, **caractérisé en ce que** le capot est muni d'au moins un filtre (20).

4. Sèche-cheveux (3) silencieux selon une des revendications 1 à 3, **caractérisé en ce qu'**il comporte une turbine (T) silencieuse, comportant un orifice d'admission de diamètre (Da) réduit de façon à diminuer le bruit généré par la turbine (T) et comportant des pales (T2) dont l'extrémité périphérique est courbée en direction du sens de rotation (R) de la turbine de façon à améliorer la pression générée par la turbine, au moins pour compenser la perte de débit résultant de la diminution du diamètre d'admission (Da) de la turbine (T).

5. Sèche-cheveux (3) silencieux selon une des revendications 1 à 4, **caractérisé en ce qu'**il comporte une goutte (G) aérodynamique disposée en amont de l'admission de la turbine (T) pour minimiser les perturbations d'air en entrée de la turbine (T) et réduire le niveau sonore du sèche-cheveux.

6. Sèche-cheveux (3) silencieux selon une des revendications 1 à 5, **caractérisé en ce que** la chambre (10) du silencieux (1) comporte au moins un filtre (15) de section annulaire remplissant sensiblement le volume compris entre le diamètre (Dc) de la chambre et le diamètre (Da) d'admission ou le diamètre (De) d'entrée et comportant au moins un matériau poreux, le volume et l'indice de porosité du filtre (15) étant accordées en fonction desdites fréquences sonores déterminées.

7. Sèche-cheveux (3) silencieux selon une des revendications 1 à 6, **caractérisé en ce que** la chambre (10) du silencieux (1) comporte plusieurs filtres (15a, 15b) de densités croissantes en direction de l'orifice (330) d'admission de la turbine (T), de section annulaire remplissant sensiblement le volume compris entre le diamètre (Dc) de la chambre et le diamètre (Da) d'admission ou le diamètre (De) d'entrée et comportant au moins un matériau poreux, le volume et les indices de porosité des filtres (15) étant accordés en fonction desdites fréquences sonores déterminées.

8. Sèche-cheveux (3) silencieux selon une des revendications 6 à 7, **caractérisé en ce que** le matériau poreux présent dans le ou les filtre(s) (15, 15a, 15b) est un matériau à alvéoles ouvertes.

9. Sèche-cheveux (3) silencieux selon une des revendications 1 à 8, **caractérisé en ce que** la chambre (10) du silencieux (1) est de section cylindrique, tronconique ou polygonale.

10. Silencieux (1) pour appareils de séchage, en particulier pour sèche-cheveux (3) comportant au moins un carter (30) comprenant une extrémité d'entrée (33), une extrémité de sortie (31) et au moins un moteur (M) entraînant en rotation au moins une turbine (T) aspirant de l'air par au moins un orifice (330) d'admission au niveau de l'extrémité d'entrée (33) en amont de la turbine (T) et l'expulsant en aval par au moins un orifice (310) de sortie au niveau de l'extrémité de sortie (31),
- le silencieux (1) comportant au moins une chambre (10) montée de façon sensiblement étanche en amont de l'orifice (330) d'admission et comportant un orifice (110) d'entrée pour l'aspiration d'air en amont de la chambre (10), le diamètre (Dc) de la chambre (10) étant supérieur au diamètre (Da) de l'orifice (330) d'admission et au diamètre (De) de l'orifice (110) d'entrée,
- le silencieux (1) étant apte à être fixé de façon amovible sur le carter (30), au niveau de l'extrémité d'entrée (33), grâce à des moyens (35) de fixation amovible sur le carter (30) identiques à des moyens (25) de fixation amovible d'un capot (2) du sèche-cheveux (3),
**caractérisé en ce que** :
- le silencieux (1) comporte, à proximité de l'orifice (110) d'entrée, des moyens d'ancrage pour les moyens (25) de fixation amovible du capot (2) du sèche-cheveux (3), de telle sorte que le silencieux (1) puisse être interposé entre le carter (30) et le capot (2) du sèche-cheveux (3).

## Patentansprüche

1. Leiser Haartrockner (3), umfassend wenigstens ein Gehäuse (30) mit einem Eintrittsende (33), einem Austrittsende (31) und wenigstens einem Motor (M), der wenigstens eine Turbine (T) in Drehung versetzt, die Luft durch wenigstens eine Einlassöffnung (330) oberhalb der Turbine (T) einsaugt und durch wenigstens eine unterhalb gelegene Austrittsöffnung (31) ausstößt, wobei der Haartrockner einen Schalldämpfer (1) umfasst, der wenigstens eine Kammer (10) umfasst, die im wesentlichen dicht oberhalb der Einlassöffnung (330) montiert ist und eine Eintrittsöffnung (110) zum Ansaugen von Luft oberhalb der Kammer (10) aufweist,
- wobei der Durchmesser (Dc) der Kammer (10) größer ist als der Durchmesser (Da) der Einlassöffnung (330) und als der Durchmesser (De) der Eintrittsöffnung (110);
**dadurch gekennzeichnet, dass**:
- der Haartrockner (3) eine Abdeckung (2) zum abnehmbaren Befestigen am Gehäuse (30) in der Höhe des Eintrittsendes (33) aufweist;
- der Schalldämpfer (1) abnehmbar am Gehäuse (30) in der Höhe des Eintrittsendes (33) dank Mitteln (35) zum abnehmbaren Befestigen am Gehäuse (30) befestigt werden kann, die mit Mitteln (25) zum abnehmbaren Befestigen der Abdeckung (2) des Haartrockners (3) identisch sind;
- der Schalldämpfer einerseits Mittel (35) zum abnehmbaren Befestigen am Gehäuse (30) umfasst, die mit den Mitteln (25) zum abnehmbaren Befestigen der Abdeckung (2) identisch sind, und andererseits in der Nähe der Eintrittsöffnung (110) Mittel zum Verankern der Mittel (25) zum Befestigen der Abdeckung (2) umfasst, so dass der Schalldämpfer zwischen das Gehäuse (30) und die Abdeckung (2) geschaltet werden kann.

2. Leiser Haartrockner (3), der wenigstens ein Gehäuse (30) mit einem Eintrittsende (33), einem Austrittsende (31) und wenigstens einem Motor (M) umfasst, der wenigstens eine Turbine (T) in Drehung versetzt, die Luft durch eine Einlassöffnung (330) oberhalb der Turbine (T) einsaugt und durch eine unterhalb gelegene Austrittsöffnung (31) ausstößt, wobei der Haartrockner einen Schalldämpfer (1) umfasst, der wenigstens eine Kammer (10) aufweist, die im Wesentlichen dicht oberhalb der Einlassöffnung (330) montiert ist und eine Eintrittsöffnung (110) zum Einsaugen von Luft oberhalb der Kammer (10) aufweist,
- wobei der Durchmesser (Dc) der Kammer (10) größer ist als der Durchmesser (Da) der Einlassöffnung (330) und als der Durchmesser (De) der Eintrittsöffnung (110);
**dadurch gekennzeichnet, dass**:
- der Haartrockner (3) eine Abdeckung (2) umfasst, die abnehmbar am Gehäuse (30) in der Höhe der Eintrittsende (33) befestigt werden kann;
- der Schalldämpfer (1) eine Abdeckung umfasst, die einstückig mit dem Schalldämpfer ausgebildet ist;
- der Schalldämpfer (1) abnehmbar am Gehäuse (30) in der Höhe des Eintrittsendes (33) anstelle der Abdeckung (2) des Haartrockners (3) mit den Mitteln (35) zum abnehmbaren Befestigen des Schalldämpfers (1) befestigt werden kann.

3. Leiser Haartrockner (3) nach einem der Ansprüche 1 bis 2, **dadurch gekennzeichnet, dass** die Abdeckung mit wenigstens einem Filter (20) versehen ist.

4. Leiser Haartrockner (3) nach einem der Ansprüche 1 bis 3, **dadurch gekennzeichnet, dass** er eine leise Turbine (T) umfasst, die eine Einlassöffnung mit einem reduzierten Durchmesser (Da) aufweist, um das von der Turbine (T) erzeugte Geräusch zu vermindern, und Flügel (T2) umfasst, deren peripheres Ende in Richtung des Drehsinns (R) der Turbine so gekrümmt ist, dass der von der Turbine erzeugte Druck verbessert wird, um wenigstens den durch die Reduzierung des Einlassdurchmessers (Da) der Turbine (T) verursachten Leistungsverlust zu kompensieren.

5. Leiser Haartrockner (3) nach einem der Ansprüche 1 bis 4, **dadurch gekennzeichnet, dass** er einen aerodynamischen Tropfen (G) umfasst, der oberhalb des Einlasses der Turbine (T) angeordnet ist, um die Luftverwirbelungen am Eingang der Turbine (T) zu minimieren und den Schallpegel des Haartrockners zu reduzieren.

6. Leiser Haartrockner (3) nach einem der Ansprüche 1 bis 5, **dadurch gekennzeichnet, dass** die Kammer (10) des Schalldämpfers (1) wenigstens ein Filter (15) mit einem ringförmigen Querschnitt umfasst, der im Wesentlichen das Volumen zwischen dem Durchmesser (Dc) der Kammer und dem Einlassdurchmesser (Da) oder dem Eingangsdurchmesser (De) ausfüllt und wenigstens ein poröses Material umfasst, wobei das Volumen und der Porösitätsindex des Filters (15) in Abhängigkeit von den bestimmten Schallfrequenzen bestimmt werden.

7. Leiser Haartrockner (3) nach einem der Ansprüche 1 bis 6, **dadurch gekennzeichnet, dass** die Kammer (10) des Schalldämpfers (1) mehrere Filter (15a, 15b) mit in Richtung der Einlassöffnung (330) der Turbine (T) zunehmenden Dichten umfasst, wobei der ringförmige Querschnitt das Volumen zwischen dem Durchmesser (Dc) der Kammer und dem Einlassdurchmesser (Da) oder dem Eingangsdurchmesser (De) im Wesentlichen ausfüllt und wenigstens ein poröses Material umfasst, wobei das Volumen und die Porösitätsindexe der Filter (15) in Abhängigkeit von den bestimmten Schallfrequenzen abgestimmt werden.

8. Leiser Haartrockner (3) nach einem der Ansprüche 6 bis 7, **dadurch gekennzeichnet, dass** das poröse Material in den ein oder mehreren Filtern (15, 15a, 15b) ein offenzelliges Material ist.

9. Leiser Haartrockner (3) nach einem der Ansprüche 1 bis 8, **dadurch gekennzeichnet, dass** die Kammer (10) des Schalldämpfers (1) einen zylindrischen, kegelstumpfförmigen oder polygonalen Querschnitt hat.

10. Schalldämpfer (1) für Trocknungsgeräte, insbesondere für Haartrockner (3), der wenigstens ein Gehäuse (30) mit einem Eintrittsende (33), einem Austrittsende (31) und wenigstens einem Motor (M) umfasst, der wenigstens eine Turbine (T) in Drehung versetzt, die Luft durch wenigstens eine Einlassöffnung (330) in der Höhe des Eintrittsendes (33) oberhalb der Turbine (T) einsaugt und unterhalb durch wenigstens eine Austrittsöffnung (310) in der Höhe des Austrittsendes (31) ausstößt,
- wobei der Schalldämpfer (1) wenigstens eine Kammer (10) umfasst, die im Wesentlichen dicht oberhalb der Einlassöffnung (330) montiert ist und eine Eintrittsöffnung (110) zum Einsaugen von Luft oberhalb der Kammer (10) aufweist, wobei der Durchmesser (Dc) der Kammer (10) größer ist als der Durchmesser (Da) der Einlassöffnung (330) und als der Durchmesser (De) der Eintrittsöffnung (110),
- wobei der Schalldämpfer (1) abnehmbar am Gehäuse (30) in der Höhe des Eintrittsendes (33) dank Mitteln (35) zum abnehmbaren Befestigen am Gehäuse (30) befestigt werden kann, die mit den Mitteln (25) zum abnehmbaren Befestigen einer Abdeckung (2) des Haartrockners (3) identisch sind, **dadurch gekennzeichnet, dass**
- der Schalldämpfer (1) in der Nähe der Eintrittsöffnung (110) Verankerungsmittel für die Mittel (25) zum abnehmbaren Befestigen der Abdeckung (2) des Haartrockners (3) umfasst, so dass der Schalldämpfer (1) zwischen das Gehäuse (30) und die Abdeckung (2) des Haartrockners (3) geschaltet werden kann.

## Claims

1. Quiet hairdryer (3) including at least a case (30) comprising an inlet end (33), an output end (31) and at least a motor (M) driving in rotation at least a turbine (T) sucking air through at least one inlet orifice (330) upstream of the turbine (T) and expelling it through at least one downstream outlet orifice (31), the hairdryer including a silencer (1) comprising at least a chamber (10) mounted in substantially sealed manner upstream of the intake orifice (330) and including an inlet orifice (110) for suction of air upstream of the chamber (10),
- the diameter (Dc) of the chamber (10) being greater than the diameter (Da) of the intake orifice (330) and than the diameter (De) of the inlet orifice (110) ;
**characterised in that**:
- the hairdryer (3) includes a cover (2) able to be fixed in detachable manner on the case (30), at the inlet end (33) ;
- the silencer (1) is able to be fixed in detachable manner on the case (30), at the inlet end (33) by means of means (35) for detachable fixing on the case (30) identical to means (25) for detachable fixing of the cover (2) of the hairdryer (3);
- the silencer includes, on the one hand, means (35) for detachable fixing on the case (30) identical to the means (25) for detachable fixing of the cover (2), and on the other, in the proximity of the inlet orifice (110), means for anchoring of the fixing means (25) of the cover (2), so that the silencer can be interposed between the case (30) and the cover (2).

2. Quiet hairdryer (3) including at least a case (30) comprising an inlet end (33), an outlet end (31) and at least a motor (M) driving in rotation at least a turbine (T) sucking air through at least one intake orifice (330) upstream of the turbine (T) and expelling it through at least one downstream outlet orifice (31), the hairdryer including a silencer (1) comprising at least one chamber (10) mounted in substantially sealed manner upstream of the intake orifice (330) and including an inlet orifice (110) for suction of air upstream of the chamber (10),
- the diameter (Dc) of the chamber (10) being greater than the diameter (Da) of the intake orifice (330) and than the diameter (De) of the inlet orifice (110);
**characterised in that**:
- the hairdryer (3) includes a cover (2) able to be fixed in detachable manner on the case (30), at the inlet end (33);
- the silencer (1) includes a cover made in one piece with the silencer;
- the silencer (1) is able to be fixed in detachable manner on the case (30), at the inlet end (33), replacing the cover (2) of the hairdryer (3) with means (35) for detachable fixing of the silencer (1).

3. Quiet hairdryer (3) in accordance with one of claims 1 to 2, **characterised in that** the cover is provided with at least one filter (20).

4. Quiet hairdryer (3) in accordance with one of claims 1 to 3, **characterised in that** it includes a silent turbine (T), including an intake orifice of small diameter (Da) so as to reduce the noise generated by the turbine (T) and including blades (T2) the peripheral end of which is curved in the direction of rotation (R) of the turbine so as to improve the pressure generated by the turbine, at least to compensate for the loss of flow resulting from the reduction of the intake diameter (Da) of the turbine (T).

5. Quiet hairdryer (3) in accordance with one of claims 1 to 4, **characterised in that** it includes an aerodynamic blister (G) arranged upstream of the intake of the turbine (T) to minimise air turbulence at the inlet of the turbine (T) and reduce the sound level of the hairdryer.

6. Quiet hairdryer (3) in accordance with one of claims 1 to 5, **characterised in that** the chamber (10) of the silencer (1) includes at least one filter (15) of annular section substantially filling the volume between the diameter (Dc) of the chamber and the intake diameter (Da) or the inlet diameter (De) and including at least a porous material, the volume and the porosity index of the filter (15) being assigned as a function of said determined sound frequencies.

7. Silent hairdryer (3) in accordance with one of claims 1 to 6, **characterised in that** the chamber (10) of the silencer (1) includes a plurality of filters (15a, 15b) of densities increasing in the direction of the intake orifice (330) of the turbine (T), of annular section substantially filling the volume between the diameter (Dc) of the chamber and the intake diameter (Da) or the inlet diameter (De) and including at least a porous material, the volume and the porosity indexes of the filters (15) being assigned as a function of said determined sound frequencies.

8. Quiet hairdryer (3) in accordance with one of claims 6 to 7, **characterised in that** the porous material present in the filter or filters (15, 15a, 15b) is an open cell material.

9. Quiet hairdryer (3) in accordance with one of claims 1 to 8, **characterised in that** the chamber (10) of the silencer (1) is of cylindrical, truncated conical or polygonal section.

10. Silencer (1) for drying apparatus, in particular for hairdryer (3) including at least a case (30) comprising an inlet end (33), an outlet end (31) and at least a motor (M) driving in rotation at least a turbine (T) sucking air through at least one intake orifice (330) at the inlet end (33) upstream of the turbine (T) and expelling it downstream through at least one outlet orifice (310) at the outlet end (31),
- the silencer (1) including at least a chamber (10) mounted in substantially sealed manner upstream of the intake orifice (330) and including an inlet orifice (110) for suction of air upstream of the chamber (10), the diameter (Dc) of the chamber (10) being greater than the diameter (Da) of the intake orifice (330) and than the diameter (De) of the inlet orifice (110),
- the silencer (1) being able to be fixed in detachable manner on the case (30), at the inlet end (33), by means of detachable fixing means (35) on the case (30) identical to detachable fixing means (25) of a cover (2) of the hairdryer (3), **characterised in that**:
- the silencer (1) includes, in the proximity of the inlet orifice (110), anchoring means for the detachable fixing means (25) of the cover (2) of the hairdryer (3) so that the silencer (1) can be interposed between the case (30) and the cover (2) of the hairdryer (3).
